Europäisches Patentamt

European Patent Office

Office européen des brevets

(19)

(11) Veröffentlichungsnummer : **0 358 601 B1**

(12) # EUROPÄISCHE PATENTSCHRIFT

(45) Veröffentlichungstag der Patentschrift :
**07.10.92 Patentblatt 92/41**

(51) Int. Cl.⁵ : **A61F 2/36,** A61F 2/30,
A61L 27/00

(21) Anmeldenummer : **89810533.3**

(22) Anmeldetag : **13.07.89**

(54) **Verankerungsschaft für eine Endoprothese.**

(30) Priorität : **30.08.88 CH 3232/88**

(43) Veröffentlichungstag der Anmeldung :
**14.03.90 Patentblatt 90/11**

(45) Bekanntmachung des Hinweises auf die
Patenterteilung :
**07.10.92 Patentblatt 92/41**

(84) Benannte Vertragsstaaten :
**AT BE DE ES FR GB IT NL SE**

(56) Entgegenhaltungen :
**EP-A- 0 145 339**
**WO-A-82/03323**
**WO-A-87/04916**
**AU-B- 480 221**
**DE-A- 2 636 644**
**DE-A- 2 751 832**
**DE-A- 2 758 541**

(56) Entgegenhaltungen :
**DE-A- 2 933 237**
**DE-A- 3 003 758**
**FR-A- 1 506 594**
**FR-A- 2 339 388**
**FR-A- 2 425 237**
**GB-A- 2 045 082**
**US-A- 4 362 681**

(73) Patentinhaber : **GEBRÜDER SULZER
AKTIENGESELLSCHAFT
Zürcherstrasse 9
CH-8401 Winterthur (CH)**

(72) Erfinder : **Wintermantel, Erich, Dr.-med.
Breitlerstrasse 1
CH-5442 Fislisbach (CH)**
Erfinder : **Flemming, Manfred, Prof. Dr.-Ing.
Rigistrasse 18
CH-5443 Niederrohrdorf (CH)**
Erfinder : **Frey, Otto, Dr.
Wallrütistrasse 56
CH-8400 Winterthur (CH)**

## Beschreibung

Die Erfindung betrifft einen an einen individuellen Patienten anpassbaren Verankerungsschaft für eine Endoprothese, bestehend aus einem festigkeitsbestimmenden Kern und einem die Oberflächenform bestimmenden Mantel, wobei der Kern aus Kunststoff und der Mantel aus einem Thermoplast bestehen.

Aus der GB-A-2 045 082 sind Verankerungsschäfte für eine Verankerung in einem Knochenzementbett bekannt, die aus einem Kern, beispielsweise aus Metall oder Kunststoff, und einem Thermoplast-Mantel bestehen, der ein- oder mehrlagig ausgeführt sein kann und einen vom Kern nach aussen abnehmenden Elastizitätsmodul hat, so dass dieser in seiner äussersten Zone möglichst an den Elastizitätsmodul des Knochenzementes und in seiner innerstes Zone möglichst an denjenigen des Kernes angeglichen ist. Die "Einstellung" der verschiedenen Elastizitätsmodulen in den einzelnen Zonen kann dabei unter anderem durch in Abmessungen und/oder Material unterschiedliches Fasermaterial erfolgen. Der Mantel dient dabei zusätzlich dafür, auch bei komplizierten Kernformen, eine einfache, meist konische, Aussenform zu erzeugen. Da diese bekannten Schäfte in ein Knochenzementbett eingebettet werden, ist bei ihnen eine Anpassbarkeit an Form und Wandoberfläche des Knochenhohlraumes nicht erforderlich.

In der DE-OS 26 36 644 werden Endoprothesen beschrieben aus einer Kombination eines körperverträglichen Kunststoffes, z.B. eines Acrylharzes oder eines anderen thermoplastischen Polymers, mit einem Verstärkungsmaterial, z.B. mit Fasern verschiedenster Art, die beispielsweise als Kurz- oder Endlosfasern vorliegen. Durch diese Kombination soll einerseits die notwendige Festigkeit der Prothese erreicht, ihr andererseits jedoch eine dem Knochematerial angepasste Steifigkeit verliehen werden. Zur guten Anpassung an den Knochen und zur Minimalisierung der Flächenpressung kann der Schaft dabei in weichem Zustand in den Knochen eingelassen werden und erst im Knochen selbst eine Aushärtung erfahren, wobei über diese Aushärtung im menschlichen Körper keine Angaben gemacht werden.

Aus der EP-B-74 981 ist eine Prothese bekannt, deren Schaft, zumindest teilweise, aus aushärtbarem Pre-Preg-Material besteht, dessen Aushärtung erst im Knochenhohlraum erfolgt, nachdem es durch aufblasbare Anpressmittel gegen die Wandung des operativ geschaffenen Hohlraumes gepresst worden ist. Auch mit dieser Prothese soll eine Anpassung der Schaftform an einen individuellen operativ geschaffenen Hohlraum im Knochen erzielt werden.

Bei diesen bekannten Schäften erfolgt eine irreversible Härtung des Kunststoff-Mantels; das Problem allfälliger Reoperationen ohne teilweise Zerstörung der Prothesenoberfläche und/oder anwachsenden Knochengewebes wird dabei nicht gelöst.

Aufgabe der Erfindung ist es, einen, an die im Einzelfall vorliegenden Gegebenheiten eines individuellen Knochens bzw. Patienten, anpassbaren Prothesenschaft zu schaffen, der Reoperationen erlaubt, ohne dass die genannten Beschädigungen seiner Oberfläche und/oder des Knochengewebes in Kauf genommen werden müssen.

Mit der Erfindung wird diese Aufgabe dadurch gelöst, dass der aus faserverstärktem Kunststoff bestehende Kern, für eine Vorbearbeitung in Form und Abmessungen vor dem Aufbringen des Mantels, mit Hilfe von unterschiedlichen Faserdichten und/oder Faserorientierungen inhomogen aufgebaut ist, wobei ein zentraler, im wesentlichen die Belastungen aufnehmender Bereich von einem mechanisch bearbeitbaren, formgebenden Bereich umgeben ist, und dass ferner der an die Begrenzung des operativ geschaffenen Knochenhohlraumes anpassbare Mantel eine Erweichungstemperatur zwischen 45°C und 60°C hat.

Eine mechanische Vorbearbeitung des "äusseren" Kernbereiches erlaubt eine weitgehende Anpassung des Kerns an den individuellen Patienten, während die niedrige Erweichungstemperatur zum eine selbst bei "rauhen" Oberflächen des Operationshohlraumes ein gutes Anschmiegen des Mantels an diese Oberflächen ermöglicht und zum anderen bei Reoperationen den Mantel so "weich" werden lässt, dass der Schaft ohne Beschädigungen aus dem Knochen entfernt werden kann.

Als Werkstoffe für den erweichenden Mantel sind alle thermoplastischen Materialien geeignet, die einen Erweichungsbereich unterhalb einem physiologisch bedenklichen Grenzwert haben. Dieser Grenzwert liegt etwa bei 60°C, wobei kurzzeitig unter Umständen auch Temperaturen bis 75°C zulässig sind. Bevorzugte thermoplastische Kunststoffe sind Methacrylate, insbesondere Polyäthylen- oder Polyisobutylen-Methacrylat. Da der Mantel - und unter Umständen auch der Kern - aus auspolymerisierten Kunststoffen bestehen hat die neue Konstruktion den zusätzlichen Vorteil, dass durch sie keine, im allgemeinen toxischen Monomere der Kunststoffe in den Körper eingetragen werden.

Auch unterscheidet sich das operative Vorgehen bei der Implantation der neuen Prothese vom Vorgehen bei der bekannten Implantation in ein Knochenzementbett. Der Mantel ist im vorliegenden Fall Bestandteil der Prothese und wird, mit dem Prothesenkern haftend verbunden, in den vorbereiteten Knochenkanal eingeführt. Im Fall der Zementierung wird der Zement als separater Werkstoff vor der Implantation der Prothese in den Knochenkanal eingebracht. In jenem Fall ergibt sich also eine Haftung zwischen Prothese und Knochenzement erst im Knochen. Diese Haftung ist damit den intraoperativen Umgebungsbedingungen unterworfen, die erheblich von Implantation zu Implantation variieren können.

Bei der Implantation wird der neue Schaft, der in seiner Dicke relativ zum Knochenhohlraum leicht überdimensioniert ist, beispielsweise zunächst geringfügig erwärmt und in den Knochenhohlraum eingesetzt, wobei in diesem Zustand plastisch verformbares überschüssiges Mantelmaterial zur Einsetzöffnung hin ausweicht, wo es entfernt werden kann.

Durch weiteres Erwärmen, beispielsweise mit Hilfe eines Heizelementes oder - falls Mantel und/oder Kern elektrisch leitend ausgebildet sind - induktiv, wird das Mantelmaterial weiter erwärmt bis es fliessfähig wird und sich an die Wandoberflächen des Knochenhohlraumes anpassen kann. Die Stabilisierung des Mantels - und gegebenenfalls, falls Kern und Mantel aus gleichem Kunststoff-Matrixmaterial bestehen auch des Kernes - erfolgt durch einfaches Abkühlen auf Körpertemperatur, wobei die Prothese gleichzeitig im Knochenhohlraum fixiert wird. Für Reoperationen wird zumindest der Mantel wieder aufgewärmt, dadurch fliessfähig und lässt sich unter plastischem Verformen aus dem Knochen ohne Beschädigungen entfernen.

Das Erweichungsintervall des Mantels zwischen 45°C und 60°C. Bestehen Kern und Mantel - wie erwähnt - aus dem gleichen Kunststoff, so unterscheiden sich Kern und Mantel durch ihre Fasern-Armierung, die beim Kern langfaserig oder orientiert kurzfaserig ist, während der Mantel - wenn überhaupt - eine regellose Kurzfaser-Verstärkung enthält.

Werden, wie erwähnt, Kern und/oder Mantel elektrisch leitend ausgebildet, so hat das den Vorteil, dass sie relativ gleichmässig über ihr ganzes Volumen induktiv erwärmt werden können.

Weiterhin ist es möglich, dass der Mantel das Knochenwachsum stimulierende Stoffe und/oder mindestens auf einem Teil seiner oberfläche anwachsendes Gewebe "festhaltende" Strukturen, wie z.B. im Kunststoff des Mantels eingebettete, geflochtene oder gewebte Drahtnetze enthält.

Weiterhin lässt sich die Implantation vereinfachen, wenn das distale Ende des Kerns ein nicht erweichendes Abschlusselement, beispielsweise aus Titan, aufweist, auf dem der Mantel abgestützt ist.

Im folgenden wird die Erfindung anhand von Ausführungsbeispielen im Zusammenhang mit der Zeichnung näher erläutert.

Fig. 1 zeigt im Längsschnitt eine in den proximalen Bereich eines Femurs implantierte Femurkopfprothese;
Fig. 2 gibt in gleicher Darstellung eine Variante von Fig. 1 wieder.

Auf einem Prothesenhals 1 ist beispielsweise mit Hilfe einer bekannten Konussteckverbindung ein Gelenkkopf 2 befestigt. Dieser besteht in den gezeigten Beispielen aus Metall oder Keramik, während der Prothesenhals 1 einen Teil eines Schaftkernes 3 bildet. Dieser ist aus faserverstärktem thermoplastischen Kunststoff - im vorliegenden Beispiel aus mit Kohlenstoff-Fasern verstärktem Polyisobutylen-Methacrylat - hergestellt und aufgrund von Computer-Tomographie-Aufnahmen an den individuellen Patienten in Form und Abmessungen angepasst.

Der Kern 3 ist inhomogen gestaltet und hat einen festigkeitsbestimmenden, zentralen Bereich 3a, der von einem mechanisch bearbeitbaren formgebenden Bereich 3b umgeben ist. Die Inhomogenität wird dabei beispielsweise durch unterschiedliche Faserndichten, unterschiedliche Faserformen - z.B. Langfasern oder Kurzfasern - und unterschiedliche Faserorientierungen - z.B. gerichtet oder regellos - erreicht.

Im vorliegenden Beispiel enthält der innere Bereich 3a durchgehende Langfasern, während der formgebende Bereich mit Kurzfasern mit Längen von 1 mm - 5 mm gleicher oder unterschiedlicher Länge, die im wesentlichen in die Richtung der Längsachse des Schaftes ausgerichtet sind, verstärkt ist. Um eine induktive Erwärmung des Schaftes zu ermöglichen, bestehen die Fasern aus Kohlenstoff bzw. sind zusätzlich zu ihnen Kupferfäden in die Kunststoffmatrix eingelagert.

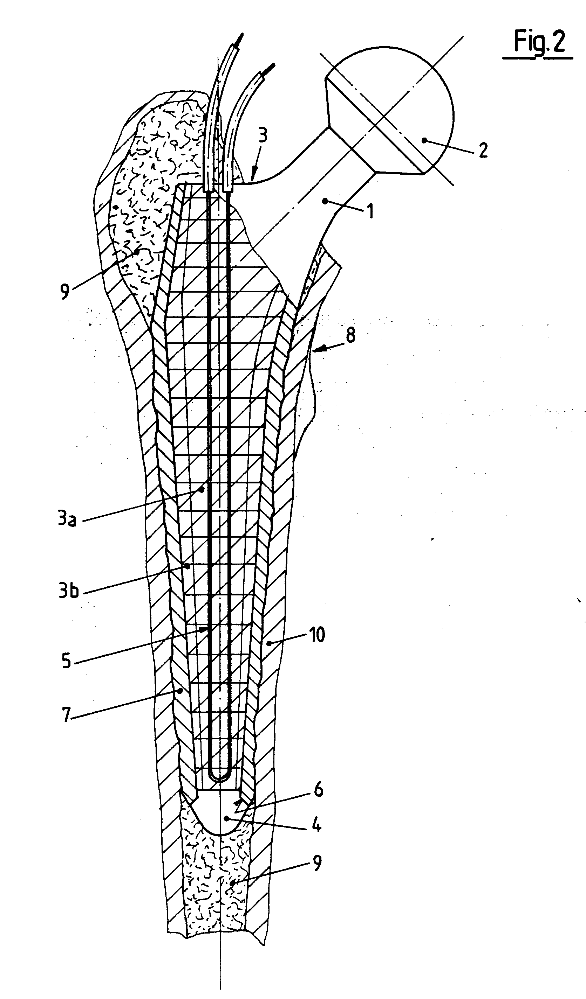

Wie in Fig. 2 gezeigt, kann in dem Bereich 3a jedoch auch eine Heizspirale 5 eingelegt sein, deren Anschlussenden für eine Spannungsquelle aus dem Prothesenhals 1 - was nicht ausdrücklich gezeigt ist - oder über das proximale Prothesenende im Bereich des grossen Trochanters herausgeführt sind.

Distal ist der Kern 3 abgeschlossen durch ein Abschlusselement 4, das nicht erweichbar ist und beispielsweise aus Titan besteht. Dieses Element 4 hat einen in Umfangsrichtung verlaufenden Absatz 6, auf dem sich ein den Kern umschliessender Mantel 7 abstützt. Dieser besteht ebenfalls aus einem thermoplastischen Kunststoff; im Gegensatz zum Kern 3 enthält er keine - oder allenfalls eine kurzfaserige regellose - Einlage aus Kohlenstoff-Fasern, wobei die Dichte der Einlage relativ gering ist.

Soll auch der Mantel 7 elektrisch leitend sein, so enthält auch er Fasern aus Kohlenstoff oder Kupferfäden.

Der Schaft ist in einen Femur 8 implantiert, in dessen spongiösem Gewebe 9 ein entsprechender Hohlraum operativ geschaffen worden ist. Durch Erwärmen und anschliessendes Erkalten hat sich der Mantel 7 an die Form des Hohlraumes bzw. an die Wandoberfläche der den Knochenhohlraum begrenzenden Kortikalis 10 angepasst, was eine innige Verbindung zwischen dem Knochen und dem Schaft ergibt.

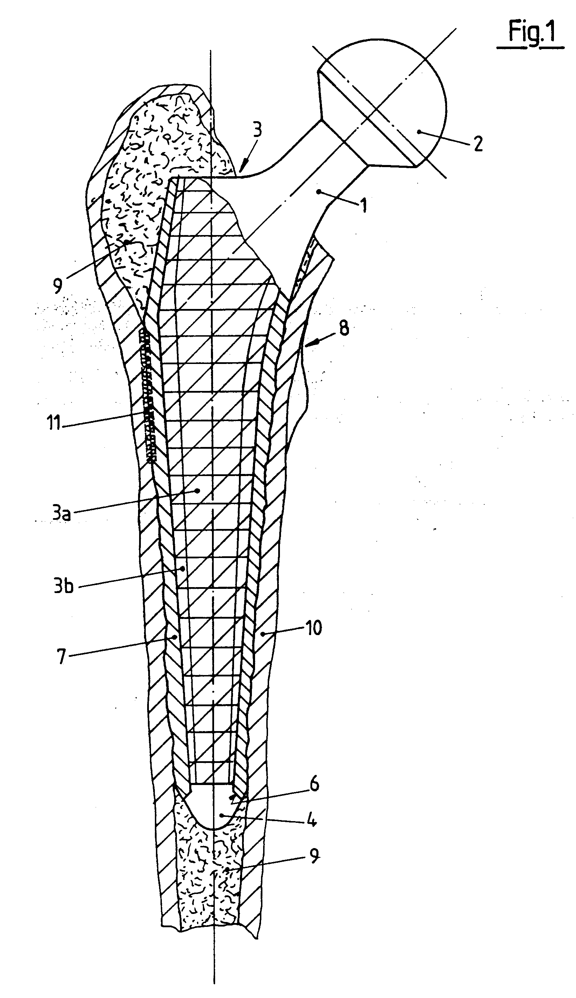

Zur Verbesserung dieser Verbindung sind Teile des Mantels mit gewobenen Metallnetzen 11 (Fig. 1) versehen, die teilweise in den Mantel 7 eingebettet sind; in ihre offenen Poren kann in bekannter Weise Knochengewebe einwachsen.

Zusätzlich kann das Anwachsen von Knochengewebe an den Mantel 7 durch Knochenwachstum stimulierende Substanzen, wie z.B. Hydroxyl-Apatit (HAP), gefördert werden, die der Kunststoffmatrix des Mantels 7 zugegeben sind.

Wird ein Kern 3 aus Metall, beispielweise aus Titan, gefertigt, so ergibt sich eine Haftung zwischen Mantel und Kern in der für die Einbettung von Implantaten in Knochenzementen bekannten Weise, wofür die Oberfläche des Metallkernes beispielsweise mit einer oberflächenvergrössernden Struktur versehen sein kann.

## Patentansprüche

1. An einen individuellen Patienten anpassbarer Verankerungsschaft für eine Endoprothese (1, 2), bestehend aus einem festigkeitsbestimmenden Kern (3) und einem die Oberflächenform bestimmenden Mantel (7), wobei der Kern (3) aus Kunststoff und der Mantel (7) aus einem Thermoplast bestehen, **dadurch gekennzeichnet,** dass der aus faserverstärktem Kunststoff bestehende Kern (3), für eine Vorbearbeitung in Form und Abmessungen vor dem Aufbringen des Mantels, mit Hilfe von unterschiedlichen Faserdichten und/oder Faserorientierungen inhomogen aufgebaut ist, wobei ein zentraler, im wesentlichen die Belastungen aufnehmender Bereich (3a) von einem mechanisch bearbeitbaren, formgebenden Bereich (3b) umgeben ist, und dass ferner der an die Begrenzung des operativ geschaffenen Knochenhohlraumes anpassbare Mantel (7) eine Erweichungstemperatur zwischen 45°C und 60°C hat.

2. Verankerungsschaft nach Anspruch 1, dadurch gekennzeichnet, dass der Mantel (7) eine mindestens annähernd konstante Dicke aufweist.

3. Verankerungsschaft nach Anspruch 1 oder 2, dadurch gekennzeichnet, dass Kern (3) und Mantel (7) aus dem gleichen Kunststoff bestehen.

4. Verankerungsschaft nach einem der Ansprüche 1 bis 3, dadurch gekennzeichnet, dass Kern (3) und/oder Mantel (7) elektrisch leitend ausgebildet sind.

5. Verankerungsschaft nach einem der Ansprüche 1 bis 4, dadurch gekennzeichnet, dass der Kunststoff-Mantel (7) das Knochenwachstum stimulierende Stoffe enthält.

6. Verankerungsschaft nach einem der Ansprüche 1 bis 5, dadurch gekennzeichnet, dass mindestens in Teilbereiche des Kunststoff-Mantels (7) mehrlagige Drahtnetze (11) eingelagert sind.

7. Verankerungsschaft nach einem der Ansprüche 1 bis 6, dadurch gekennzeichnet, dass das distale Ende des Kerns (3) ein nicht erweichendes Abschlusselement (4) aufweist, auf dem der Mantel (7) abgestützt ist.

## Claims

1. An anchoring stem, adaptable to an individual patient, for an endoprosthesis (1, 2), comprising a strength-determining core (3) and a surface-shape-determining covering (7), the core (3) being of synthetic plastic material and the covering (7) of a thermoplast, characterised in that the core (3), which is of fibre-reinforced synthetic plastic material, is non-homogeneously constructed with the aid of differing fibre densities and/or Fibre orientations for pre-working in shape and dimensions before application of the covering, a central, substantially load bearing zone (3a) being surrounded by a mechanically workable, shape-determining zone (3b), and in addition the covering (7), which can be adapted to the boundary of the surgically created bone cavity, has a softening temperature between 45°C and 60°C.

2. An anchoring stem as claimed in claim 1, characterised in that the covering (7) is of at least substantially constant thickness.

3. An anchoring stem as claimed in claim 1 or 2, characterised in that the core (3) and covering (7) are made from the same synthetic plastic material.

4. An anchoring stem as claimed in any of claims 1 to 3, characterised in that the core (3) and/or covering (7) are electrically conductive.

5. An anchoring stem as claimed in any of claims 1 to 4, characterised in that the synthetic plastics covering (7) contains substances for stimulating bone growth.

6. An anchoring stem as claimed in any of claims 1 to 5, characterised in that multi-layer wire nets (11) are embedded at least in portions of the synthetic plastics covering (7).

7. An anchoring stem as claimed in any of claims 1 to 6, characterised in that the distal end of the core (3) has a non-softening closure element (4) on which the covering (7) is supported.

**Revendications**

1. Tige d'ancrage, pouvant être adaptée individuellement à un patient, pour une endoprothèse (1, 2), constituée d'un noyau (3) déterminant la résistance et d'une enveloppe (7) déterminant la forme de la surface, le noyau (3) étant en matière synthétique et l'enveloppe (7) en matière thermoplastique, caractérisée en ce que le noyau (3) en matière synthétique renforcée par des fibres est de constitution rendue non homogène à l'aide de fibres de densité et/ou d'orientation différentes, pour un pré-usinage de sa forme et de ses dimensions avant la mise en place de l'enveloppe, une zone (3a) centrale, absorbant essentiellement les contraintes, étant entourée par une zone (3b) usinable mécaniquement, conférant la forme, et en ce qu'en outre l'enveloppe (7), adaptable aux limites de la cavité osseuse réalisée au cours d'une opération, présente une température de ramollissement comprise entre 45° et 60°C.

2. Tige d'ancrage selon la revendication 1, caractérisée en ce que l'enveloppe (7) présente une épaisseur au moins à peu près constante.

3. Tige d'ancrage selon la revendication 1 ou 2, caractérisée en ce que le noyau (3) et l'enveloppe (7) sont réalisés dans la même matière synthétique.

4. Tige d'ancrage selon l'une des revendications 1 à 3, caractérisée en ce que le noyau (3) et/ou l'enveloppe (7) sont électriquement conducteurs.

5. Tige d'ancrage selon l'une des revendications 1 à 4, caractérisée en ce que l'enveloppe (7) en matière synthétique contient des substances stimulant la croissance osseuse.

6. Tige d'ancrage selon l'une des revendications 1 à 5, caractérisée en ce que des filets métalliques (11) à plusieurs couches sont noyés dans au moins des zones partielles de l'enveloppe (7) en matière synthétique.

7. Tige d'ancrage selon l'une des revendications 1 à 6, caractérisée en ce que l'extrémité distale du noyau (3) présente un élément de fermeture (4) ne se ramollissant pas, sur lequel prend appui l'enveloppe (7).

Fig.1

Fig.2